# EUROPÄISCHE PATENTSCHRIFT

(11) **EP 1 124 824 B1**
(45) Veröffentlichungstag und Bekanntmachung des Hinweises auf die Patenterteilung: **22.08.2007**
(21) Anmeldenummer: 99952566.0
(22) Anmeldetag: 14.10.1999
(51) Int. Cl.: C07D 405/12, A61K 31/35

(54) **CHROMENON- UND CHROMANONDERIVATE ALS INTEGRINHEMMER**
CHROMENONE AND CHROMANONE DERIVATIVES AS INTEGRIN INHIBITORS
DERIVES DE CHROMENONE ET DE CHROMANONE UTILISES COMME INHIBITEURS D'INTEGRINE

(30) Priorität: 30.10.1998 DE 19850131
(43) Veröffentlichungstag der Anmeldung: 22.08.2001
(73) Patentinhaber: Merck Patent GmbH, 64271 Darmstadt (DE)
(72) Erfinder: FITTSCHEN, Claus, D-64407 Fränkisch-Crumbach (DE); GOODMAN, Simon, D-64286 Darmstadt (DE); MÄRZ, Joachim, D-64521 Gross-Gerau (DE); RADDATZ, Peter, D-64665 Alsbach (DE); WIESNER, Matthias, D-55128 Mainz (DE)
(86) Internationale Anmeldenummer: PCT/EP1999/007725
(87) Internationale Veröffentlichungsnummer: WO 2000/026212

(56) Entgegenhaltungen:
- EP-A- 0 341 104
- WO-A-90/06921
- WO-A-91/19707
- WO-A-96/22288
- FR-A- 2 693 196
- FR-A- 2 717 479
- US-A- 5 703 075

## Beschreibung

Die Erfindung betrifft Verbindungen ausgewählt aus der Gruppe
a) (2S)-3-{2-[3-(1*H*-Imidazol-2-ylamino)propyl]-4-oxo-4*H*-chromen-6-yl}-2-(2,2-dimethyl-propoxycarboxamido)-propionsäure-ethylester,
b) (2S)-3-{2-[3-(1*H*-Imidazol-2-ylamino)propyl]-4-oxo-4*H*-chromen-6-yl}-2-(2,2-dimethyl-propoxycarboxamido)-propionsäure,
c) (2S)-3-{2-[3-(Pyridin-2-ylamino)propyl]-4-oxo-4*H*-chromen-6-yl}-2-(2,2-dimethyl-propoxycarboxamido)-propionsäure,
d) (2S)-3-{2-[3-(1*H*-Benzimidazol-2-ylamino)propyl]-4-oxo-4*H*-chromen-6-yl}-2-(2,2-dimethyl-propoxycarboxamido)-propionsäureethylester,
e) (2S)-3-{2-[3-(1*H*-Benzimidazol-2-ylamino)propyl]-4-oxo-4*H*-chromen-6-yl}-2-(2,2-dimethyl-propoxycarboxamido)-propionsäure,
f) (2S)-3-{2-[3-(Pyridin-2-ylamino)propyl]-4-oxo-4*H*-chromen-6-yl}-2-butylsulfonamido-propionsäure,
g) (2S)-3-{2-[3-(Pyridin-2-ylamino)propyl]-4-oxo-4*H*-chromen-6-yl}-2-benzylsulfonamido-propionsäure,
sowie deren physiologisch unbedenklichen Salze und Solvate.

Ähnliche Verbindungen sind z. B. aus WO 94/29273, WO 96/00730 und WO 96/18602 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die erfindungsgemäßen Verbindungen und ihre Salze und Solvate bei guter Verträglichkeit sehr wertvolle pharmakologische Eigenschaften besitzen. Vor allem wirken sie als Integrin-Inhibitoren, wobei sie insbesondere die Wechselwirkungen der αᵥ-Integrin-Rezeptoren mit Liganden hemmen. Besondere Wirksamkeit zeigen die Verbindungen im Fall der Integrine αᵥβ₃ und αᵥβ₅. Ganz besonders wirksam sind die Verbindungen als Adhäsionsrezeptor-Antagonisten für den Vitronectin-Rezeptor αᵥβ₃.
Diese Wirkung kann z.B. nach der Methode nachgewiesen werden, die von J.W. Smith et al. in J. Biol. Chem. 265, 11008-11013 und 12267-12271 (1990) beschrieben wird.
B. Felding-Habermann und D.A. Cheresh beschreiben in Curr. Opin. Cell. Biol. 5, 864 (1993) die Bedeutungen der Integrine als Adhäsionsrezeptoren für die unterschiedlichsten Phänomene und Krankheitsbilder, speziell in Bezug auf den Vitronectinrezeptor αᵥβ₃.

Die Abhängigkeit der Entstehung von Angiogenese von der Wechselwirkung zwischen vaskulären Integrinen und extrazellulären Matrixproteinen ist von P.C. Brooks, R.A. Clark und D.A. Cheresh in Science 264, 569-71 (1994) beschrieben.

Die Möglichkeit der Inhibierung dieser Wechselwirkung und damit zum Einleiten von Apoptose (programmierter Zelltod) angiogener vaskulärer Zellen durch ein cyclisches Peptid ist von P.C. Brooks, A.M. Montgomery, M. Rosenfeld, R.A. Reisfeld, T.-Hu, G. Klier und D.A. Cheresh in Cell 79, 1157-64 (1994) beschrieben.

Der experimentelle Nachweis, daß auch die erfindungsgemäßen Verbindungen die Anheftung von lebenden Zellen auf den entsprechenden Matrixproteinen verhindern und dementsprechend auch die Anheftung von Tumorzellen an Matrixproteine verhindern, kann in einem Zelladhäsionstest erbracht werden, der analog der Methode von F. Mitjans et al., J. Cell Science 108, 2825-2838 (1995) durchgeführt wird.

P.C. Brooks et al. beschreiben in J. Clin. Invest. 96, 1815-1822 (1995) αᵥβ₃ -Antagonisten zur Krebsbekämpfung und zur Behandlung tumorinduzierter angiogener Krankheiten.
Die erfindungsgemäßen Verbindungen können daher als Arzneimittelwirkstoffe insbesondere zur Behandlung von Tumorerkrankungen, Osteoporosen, osteolytischen Erkrankungen sowie zur Unterdrückung der Angiogenese eingesetzt werden.

Die erfindungsgemäßen Verbindungen, die die Wechselwirkung von Integrinrezeptoren und Liganden, wie z. B. von Fibrinogen an den Fibrinogenrezeptor (Glycoprotein IIb/IIIa) blockieren, verhindern als GPIIb/IIIa-Antagonisten die Ausbreitung von Tumorzellen durch Metastase. Dies wird durch folgende Beobachtungen belegt: Die Verbreitung von Tumorzellen von einem lokalen Tumor in das vaskuläre System erfolgt durch die Bildung von Mikroaggregaten (Mikrothromben) durch Wechselwirkung der Tumorzellen mit Blutplättchen. Die Tumorzellen sind durch den Schutz im Mikroaggregat abgeschirmt und werden von den Zellen des Immunsystems nicht erkannt.

Die Mikroaggregate können sich an Gefäßwandungen festsetzen, wodurch ein weiteres Eindringen von Tumorzellen in das Gewebe erleichtert wird. Da die Bildung der Mikrothromben durch Fibrinogenbindung an die Fibrinogenrezeptoren auf aktivierten Blutplättchen vermittelt wird, können die GPIIa/IIIb-Antagonisten als wirksame Metastase-Hemmer angesehen werden.

Die erfindungsgemäßen Verbindungen hemmen neben der Bindung von Fibrinogen, Fibronectin und des Willebrand-Faktors an den Fibrinogenrezeptor der Blutplättchen auch die Bindung weiterer adhäsiver Proteine, wie Vitronectin, Kollagen und Laminin, an die entsprechenden Rezeptoren auf der Oberflache verschiedener Zelltypen. Sie verhindern insbesondere die Entstehung von Blutplättchenthromben und können daher zur Behandlung von Thrombosen, Apoplexie, Herzinfarkt, Entzündungen und Arteriosklerose eingesetzt werden.

Die Eigenschaften der Verbindungen können auch nach Methoden nachgewiesen werden, die in der EP-A1-0 462 960 beschrieben sind. Die Hemmung der Fibrinogenbindung an den Fibrinogenrezeptor kann nach der Methode nachgewiesen werden, die in der EP-A1-0 381 033 angegeben ist.

Die thrombozytenaggregationshemmende Wirkung läßt sich in vitro nach der Methode von Born (Nature 4832, 927-929, 1962) nachweisen.

Gegenstand der Erfindung sind demgemäß die Verbindungen nach Anspruch 1 und ihre physiologisch unbedenklichen Salze und Solvate als GPIIb/IIIa-Antagonisten zur Bekämpfung von Thrombosen, Herzinfarkt, koronaren Herzerkrankungen und Arteriosklerose.

Gegenstand der Erfindung sind weiterhin die Verbindungen nach Anspruch 1 und ihre physiologisch unbedenklichen Salze und Solvate zur Herstellung eines Arzneimittels zur Verwendung als Integrin-Inhibitoren. Gegenstand der Erfindung sind insbesondere Verbindungen nach Anspruch 1 und ihre unbedenklichen Salze und Solvate, zur Herstellung eines Arzneimittels zur Bekämpfung von pathologisch angiogenen Erkrankungen, Tumoren, Osteoporose, Entzündungen und Infektionen.

Die erfindungsgemäßen Verbindungen können als Arzneimittelwirkstoffe in der Human- und Veterinärmedizin eingesetzt werden, zur Prophylaxe und/oder Therapie von Thrombose, myocardialem Infarkt, Arteriosklerose, Entzündungen, Apoplexie, Angina pectoris, Tumorerkrankungen, osteolytischen Krankheiten wie Osteoporose, pathologisch angiogenen Krankheiten wie z. B. Entzündungen, ophthalmologischen Krankheiten, diabetischer Retinopathie, makularer Degeneration, Myopia, okularer Histoplasmose, rheumatischer Arthritis, Osteoarthritis, rubeotischem Glaukom, ulcerativer Colitis, Morbus Crohn, Atherosklerose, Psoriasis, Restenose nach Angioplastie, viraler Infektion, bakterieller Infektion, Pilzinfektion, bei akutem Nierenversagen und bei der Wundheilung zur Unterstützung der Heilungsprozesse.

Die Verbindungen können als antimikrobiell wirkende Substanzen bei Operationen eingesetzt werden, wo Biomaterialien, Implantate, Katheter oder Herzschrittmacher verwendet werden. Dabei wirken sie antiseptisch. Die Wirksamkeit der antimikrobiellen Aktivität kann durch das von P.Valentin-Weigund et al., in Infection and Immunity, 2851-2855 (1988) beschriebene Verfahren nachgewiesen werden.

In die erfindungsgemäßen Verbindungen sind auch Solvate der Verbindungen eingeschlossen. Darunter werden Additionsverbindungen mit z.B. Wasser (Hydrate) oder Alkoholen wie Methanol oder Ethanol verstanden.

Die vor- und nachstehend aufgeführten Abkürzungen stehen für:
- Ac: Acetyl
- BOC: tert.-Butoxycarbonyl
- CBZ oder Z: Benzyloxycarbonyl
- DCCI: Dicyclohexylcarbodiimid
- DMF: Dimethylformamid
- DOPA: (3,4-Dihydroxyphenyl)-alanin
- DPFN: 3,5-Dimethylpyrazol-1-formamidinium-nitrat
- DMAP: Dimethylaminopyridin
- EDCI: N-Ethyl-N,N'-(dimethylaminopropyl)-carbodiimid
- Et: Ethyl
- Fmoc: 9-Fluorenylmethoxycarbonyl
- HOBt: 1-Hydroxybenzotriazol
- Me: Methyl
- MTB-Ether: Methyl-tert.-butylether
- Mtr: 4-Methoxy-2,3,6-trimethylphenyl-sulfonyl
- HONSu: N-Hydroxysuccinimid
- Np: Neopentyl
- OBn: Benzylester
- OBut: tert.-Butylester
- Oct: Octanoyl
- OMe: Methylester
- OEt: Ethylester
- Orn: Ornithin
- POA: Phenoxyacetyl
- TBTU: O-(Benzotriazol-1-yl)-N,N,N,N-tetramethyluroniumtetrafluoroborat
- TFA: Trifluoressigsäure
- pTSS-Salz: para-Toluolsulfonsäuresalz
- Trt: Trityl (Triphenylmethyl)
- Z oder CBZ: Benzyloxycarbonyl.

Die erfindungsgemäßen Verbindungen und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den erfindungsgemäßen Verbindungen umsetzt.

Die erfindungsgemäßen Verbindungen können vorzugsweise erhalten werden, indem man die Verbindungen aus einem ihrer funktionellen Derivate durch Behandeln mit einem solvolysierenden oder hydrogenolysierenden Mittel in Freiheit setzt.

Bevorzugte Ausgangsstoffe für die Solvolyse bzw. Hydrogenolyse sind solche, die anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, insbesondere solche, die anstelle einer HN-Gruppe eine R'-N-Gruppe tragen, worin R' eine Aminoschutzgruppe bedeutet, und/ oder solche, die anstelle des H-Atoms einer Hydroxygruppe eine Hydroxyschutzgruppe tragen, z.B. solche, die jedoch anstelle einer Gruppe -COOH eine Gruppe -COOR" tragen, worin R" eine Hydroxyschutzgruppe bedeutet.

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC, 2-lodethoxycarbonyl; Aralkyloxycarbonyl wie CBZ ("Carbobenzoxy"), 4-Methoxybenzyloxycarbonyl, FMOC; Arylsulfonyl wie Mtr. Bevorzugte Aminoschutzgruppen sind BOC und Mtr, ferner CBZ, Fmoc, Benzyl und Acetyl.

Die Abspaltung der Aminoschutzgruppe gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die Gruppen BOC, OBut und Mtr können z. B. bevorzugt mit TFA in Dichlormethan oder mit etwa 3 bis 5n HCl in Dioxan bei 15-30° abgespalten werden, die FMOC-Gruppe mit einer etwa 5- bis 50 %igen Lösung von Dimethylamin, Diethylamin oder Piperidin in DMF bei 15-30°.

Hydrogenolytisch entfernbare Schutzgruppen (z. B. CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10 %igem Pd/C in Methanol oder mit Ammoniumformiat (anstelle von Wasserstoff) an Pd/C in Methanol/DMF bei 20-30°.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat, Wasser oder Gemische der genannten Lösungsmittel.

Die Umwandlung einer Cyangruppe in eine Amidinogruppe erfolgt durch Umsetzung mit z.B. Hydroxylamin und anschließender Reduktion des N-Hydroxyamidins mit Wasserstoff in Anwesenheit eines Katalysators wie z.B. Pd/C.
Ferner ist es möglich, eine konventionelle Aminoschutzgruppe durch Wasserstoff zu ersetzen, indem die Schutzgruppe, wie oben beschrieben, solvolytisch oder hydrogenolytisch abgespalten wird oder daß man eine durch eine konventionelle Schutzgruppe geschützte Aminogruppe durch Solvolyse oder Hydrogenolyse in Freiheit setzt.

Zur Herstellung von erfindungsgemäßen Verbindungen kann man eine entsprechende Aminoverbindung mit einem amidinierenden Mittel behandeln. Als amidinierendes Mittel ist 1-Amidino-3,5-dimethylpyrazol (DPFN) bevorzugt, das insbesondere in Form seines Nitrats eingesetzt wird. Man arbeitet zweckmäßig unter Zusatz einer Base wie Triethylamin oder Ethyl-diisopropylamin in einem inerten Lösungsmittel oder Lösungsmittelgemisch, z.B. Wasser/Dioxan bei Temperaturen zwischen 0 und 120 °C, vorzugsweise zwischen 60 und 120 °C.

Zur Herstellung eines erfindungsgemäßen Amidins kann man an ein Nitril Ammoniak anlagern. Die Anlagerung erfolgt bevorzugt mehrstufig, indem man in an sich bekannter Weise a) das Nitril mit H₂S in ein Thioamid umwandelt, das mit einem Alkylierungsmittel, z.B. CH₃l, in den entsprechenden S-Alkyl-imidothioester übergeführt wird, welcher seinerseits mit NH₃ zum Amidin reagiert, b) das Nitril mit einem Alkohol, z.B. Ethanol in Gegenwart von HCl in den entsprechenden Imidoester umwandelt und diesen mit Ammoniak behandelt, oder c) das Nitril mit Lithium-bis-(trimethylsilyl)-amid umsetzt und das Produkt anschließend hydrolysiert.

Ferner kann man freie Aminogruppen in üblicher Weise mit einem Säurechlorid oder -anhydrid acylieren oder mit einem unsubstituierten oder substituierten Alkylhalogenid alkylieren, zweckmäßig in einem inerten Lösungsmittel wie Dichlormethan oder THF und /oder in Gegenwart einer Base wie Triethylamin oder Pyridin bei Temperaturen zwischen -60 und +30°.

Eine erfindungsgemäße Base kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und Disulfonsäuren, Laurylschwefelsäure. Salze mit physiologisch nicht unbedenklichen Säuren, z.B. Pikrate, können zur Isolierung und /oder Aufreinigung der erfindungsgemäßen Verbindungen verwendet werden.

Andererseits kann eine erfindungsgemäße Säure durch Umsetzung mit einer Base in eines ihrer physiologisch unbedenklichen Metall- oder Ammoniumsalze übergeführt werden. Als Salze kommen dabei insbesondere die Natrium-, Kalium-, Magnesium-, Calcium- und Ammoniumsalze in Betracht, ferner substituierte Ammoniumsalze, z. B. die Dimethyl-, Diethyl- oder Diisopropyl-ammoniumsalze, Monoethanol-, Diethanol- oder Diisopropylammoniumsalze, Cyclohexyl-, Dicyclohexylammoniumsalze, Dibenzylethylendiammoniumsalze, weiterhin z. B. Salze mit Arginin oder Lysin.

Die erfindungsgemäßen Verbindungen enthalten ein oder mehrere chirale Zentren und können daher in racemischer oder in optisch-aktiver Form vorliegen. Erhaltene Racemate können nach an sich bekannten Methoden mechanisch oder chemisch in die Enantiomeren getrennt werden. Vorzugsweise werden aus dem racemischen Gemisch durch Umsetzung mit einem optisch aktiven Trennmittel Diastereomere gebildet. Als Trennmittel eignen sich z.B. optisch aktive Säuren, wie die D- und L-Formen von Weinsäure, Diacetylweinsäure, Dibenzoylweinsäure, Mandelsäure, Äpfelsäure, Milchsäure oder die verschiedenen optisch aktiven Camphersulfonsäuren wie β-Camphersulfonsäure. Vorteilhaft ist auch eine Enantiomerentrennung mit Hilfe einer mit einem optisch aktiven Trennmittel (z.B. Dinitrobenzoyl-phenylglycin) gefüllten Säule; als Laufmittel eignet sich z.B. ein Gemisch Hexan/Isopropanol/Acetonitril, z.B. im Volumenverhältnis 82:15:3.

Natürlich ist es auch möglich, optisch aktive erfindungsgemäße Verbindungen nach den oben beschriebenen Methoden zu erhalten, indem man Ausgangsstoffe verwendet, die bereits optisch aktiv sind.

Gegenstand der Erfindung ist ferner die Verwendung der erfindungsgemäßen Verbindungen und/oder ihrer physiologisch unbedenklichen Salze zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner pharmazeutische Zubereitungen, enthaltend mindestens eine erfindungsgemäße Verbindung und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale, topische Applikation oder für eine Applikation in Form eines Inhalation-Sprays eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder mehrere weitere Wirkstoffe enthalten, z. B. ein oder mehrere Vitamine.
Für die Applikation als Inhalationsspray können Sprays verwendet werden, die den Wirkstoff entweder gelöst oder suspendiert in einem Treibgas oder Treibgasgemisch (z. B. CO₂ oder Fluorchlorkohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z. B. Ethanol. Inhalationslösungen können mit Hilfe üblicher Inhalatoren verabreicht werden.

Gegenstand der Erfindung ist auch die Verwendung der erfindungsgemäßen Verbindungen als therapeutische Wirkstoffe.

Die erfindungsgemäßen Verbindungen und ihre physiologisch unbedenklichen Salze können als Integrininhibitoren bei der Bekämpfung von Krankheiten, insbesondere von pathologisch angiogenen Erkrankungen, Thrombosen, Herzinfarkt, koronaren Herzerkrankungen, Arteriosklerose, Tumoren, Entzündungen und Infektionen verwendet werden.

Dabei können die erfindungsgemäßen Substanzen in der Regel in Analogie zu anderen bekannten, im Handel befindlichen Peptiden, insbesondere aber in Analogie zu den in der US-A-4 472 305 beschriebenen Verbindungen verabreicht werden, vorzugsweise in Dosierungen zwischen etwa 0,05 und 500 mg, insbesondere zwischen 0,5 und 100 mg pro Dosierungseinheit verabreicht. Die tägliche Dosierung liegt vorzugsweise zwischen etwa 0,01 und 2 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die parenterale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation.

Massenspektrometrie (MS): El (Elektronenstoß-Ionisation) M⁺ FAB (Fast Atom Bombardment) (M+H)⁺

### Beispiel 1

### (2S)-3-{2-[3-(1H-Imidazol-2-ylamino)propyl]-4-oxo-4H-chromen-6-yl}-2-(2,2-dimethyl-propoxycarboxamido)-propionsäure:

Die Synthese der Verbindung erfolgt z.B. wie in Schema 1 angegeben.

80 g (0.31 mol) 3-Acetyl-L-tyrosin werden in 1 I wasserfreiem Ethanol suspendiert und bei 80°C 12 h unter Rückfluß in Gegenwart von 70 g (0.37 mol) Toluol-4-sulfonsäure gekocht. Nach dem Abkühlen auf RT werden 500 ml MTB-Ether zugegeben, die ausfallenden Kristalle abgesaugt und mit MTB-Ether nachgewaschen und getrocknet.
Ausbeute: 99.4 g 3-Acetyl-L-tyrosin-ethylester ("AB") als pTSS-Salz.

20 g (47.2 mmol) "AB" werden in 320 ml Wasser und 160 ml THF suspendiert und portionsweise unter Rühren mit 8 g (94 mmol) NaHCO₃ versetzt.

Dann tropft man eine Lösung von 8.6 g (56 mmol) Chlorameisensäureneopentylester in 160 ml THF zu und rührt 30 min bei RT und arbeitet wie üblich auf. Der Rückstand wird aus MTB-Ether umkristallisiert.
Ausbeute: 16.1 g (93%) N-(2,2-Dimethylpropyl-oxycarbonyl)-3-acetyl-L-tyrosin-ethylester ("AC").

5 g (14.2 mmol) "AC" und 3.3 g (17 mmol) 4-Benzyloxy-buttersäure werden in 100 ml DMF gelöst und bei RT mit 3.1 ml (28.4 mmol) N-Methymorpholin und 4.08 g (21.3 mmol) EDCI versetzt. Nach 5 h gibt man die Reaktionslösung auf 700 ml Wasser und arbeitet wie üblich auf. Ausbeute: 7.4 g 4-Benzyloxy-buttersäure-(2-acetyl-4-(2-carboxyethyl-2-(2,2-dimethylpropyl)oxycarbonylamino-ethyl)-phenylester ("AD").

6.2 g (11.4 mmol) "AD" werden in 100 ml wasserfreiem THF gelöst und bei RT mit 342 mg (11.4 mmol) Natriumhydrid (80% in Mineralöl) gerührt. Nach 30 min wird die Lösung wird mit saurem lonentauscher neutralisiert und eingeengt.
Ausbeute: 6.2 g (2S)-3-(2-Hydroxy-2-(3-benzyloxy-propyl)-4-oxo-chroman-6-yl)-2-(2,2-dimethylpropyl)oxycarbonylamino-propionsäureethylester ("AE").

Zu einer Lösung von 6.2 g (11.4 mmol) "AE" in 180 ml Dichlormethan gibt man 18 ml Trifluoressigsäure und rührt bei RT über Nacht. Die Lösung wird anschließend eingeengt, noch 3 mal mit je 50 ml Toluol eingeengt und an Kieselgel mit Toluol/Methanol 20/1 als Eluentem chromatographiert.
Ausbeute: 4.2 g (2S)-3-(2-(3-benzyloxy-propyl)-4-oxo-4*H*-chromen-6-yl)-2-(2,2-dimethylpropyl)oxycarbonylamino-propionsäureethylester ("AF"), FAB 534.

3.5 g (6.7 mmol) "AF" werden in 50 ml Ethanol in Gegenwart von 350 mg Palladium (10% auf Aktivkohle) 1 h bei RT und Normaldruck hydriert. Nach dem Abfiltrieren des Katalysators und Einengen der Lösung fällt das Produkt als farblose, amorphe Masse an.
Ausbeute: 2.6 g (2S)-3-(2-(3-Hydroxypropyl)-4-oxo-4*H*-chromen-6-yl)-2-(2,2-dimethylpropyl)oxycarbonylamino-propionsäureethylester ("AG"), FAB 434.

Zu einer Lösung von 500 mg (1.15 mmol) "AG" und 357 mg (1.38 mmol) 2-(2,2,2-trichlor-ethoxycarbonyl)-amino-1*H*-imidazol in 20 ml wasserfreiem THF gibt man 0.267 ml (1.72 mmol) DEAD (Diethylazadicarboxylat, Azodicarbonsäure-diethylester) und 450 mg (172 mmol) Triphenylphosphin zu und rührt über Nacht bei 60°C. Die Lösung wird anschließend eingeengt und der Rückstand an Kieselgel RP-8 mit Methanol/Wasser 2:1 chromatographiert.
Ausbeute:560 mg (2S)-3-(2-(3-((1H-imidazol-2-yl)-(2,2,2-trichlor-ethoxycarbonyl)-amino)-propyl)-4-oxo-4*H* chromen-6-yl)-2-(2,2-dimethylpropyl)-oxycarbonylamino-propionsäureethylester ("AH") als farbloses Öl, FAB 675.

Eine Lösung von 280 mg (4.15 mmol) "AH" in 5 ml THF mit 0.5 ml Essigsäure und 0.5 ml Wasser wird mit 500 mg (7.7 mmol) Zinkstaub versetzt und 1 h bei RT gerührt. Anschließend filtriert man ab, engt die Lösung ein und trocknet den Rückstand.
Ausbeute: 210 mg (2S)-3-{2-[3-(1*H*-lmidazol-2-ylamino)propyl]-4-oxo-4*H-*chromen-6-yl}-2-(2,2-dimethyl-propoxycarboxamido)-propionsäureethylester ("Al"), FAB 499.

200 mg (0.4 mmol) "Al" werden in 4 ml Dioxan gelöst und mit 2 ml 1 N HCl 12 h bei 75°C gerührt. Man engt man die Lösung ein und reinigt den Rückstand durch präparative HPLC an RP-18 Kieselgel mit einem Laufmittelgradienten (Wasser/Acetonitril 99:1 nach 1:99 in 60 min). Das Produkt fällt nach Gefriertrocknung der HPLC-Fraktionen als weißes, amorphes Pulver an.
Ausbeute: 103 mg (2S)-3-{2-[3-(1*H*-Imidazol-2-ylamino)propyl]-4-oxo-4*H-*chromen-6-yl}-2-(2,2-dimethyl-propoxycarboxamido)-propionsäure ("AK") F. 105-110°; FAB 471.

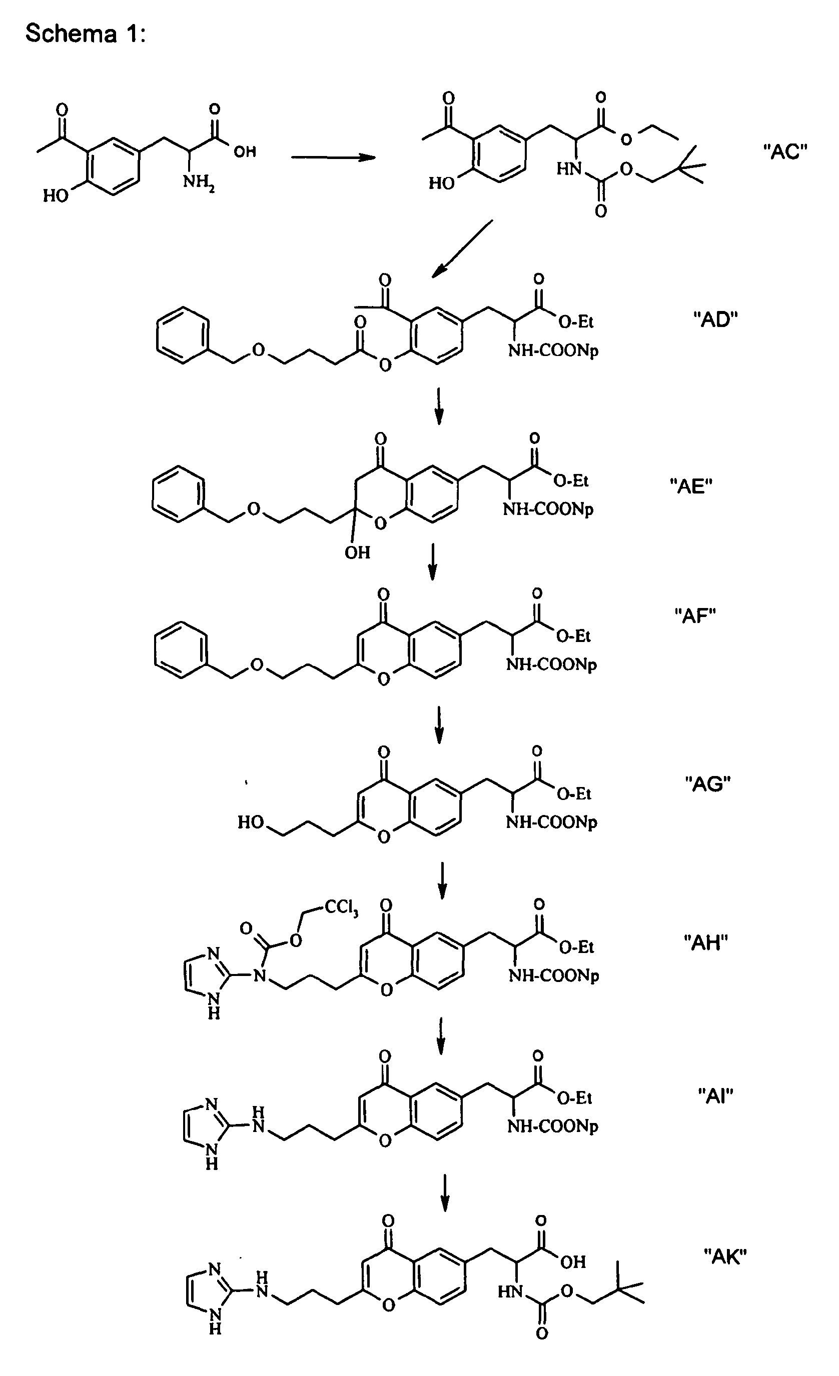

### Beispiel 2

### (2S)-3-{2-[3-(Pyridin-2-ylamino)propyl]-4-oxo-4H-chromen-6-yl}-2-(2,2-dimethyl-propoxycarboxamido)-propionsäure:

Die Synthese der Verbindung erfolgt z.B. wie in Schema 2 angegeben.

Eine Lösung von 0.5 g (1.37 mmol) "AC" und 630 mg (1.77 mmol) 4-(Pyridin-2-yl-(2,2,2-trichlor-ethoxycarbonyl)-amino)-buttersäure in 10 ml Dichlormethan wird bei RT mit 420 mg (2.04 mmol) DCC und 20 mg DMAP versetzt und 15 h gerührt. Dann filtriert man vom ausgefallenen Dicyclohexylharnstoff ab, wäscht den Rückstand mit Dichlormethan nach und engt die Lösung ein. Der Rückstand wird an Kieselgel mit Toluol/Aceton 20:1 chromatographiert.
Ausbeute: 130 mg 4-(Pyridin-2-yl-(2,2,2-trichlor-ethoxycarbonyl)-amino)-buttersäure-(2-acetyl-4-(2-carboxyethyl-2-(2,2-dimethylpropyl)oxycarbonylamino-ethyl)-phenylester ("BB"), FAB 704.

130 mg (0.185 mmol) "BB" werden mit 5.4 mg (0.18 mmol) Natriumhydrid (80% in Mineralöl) in 5 ml THF bei RT umgesetz. Nach 45 min neutralisiert man mit Essigsäure und engt zum Rückstand ein.
Ausbeute: 130 mg (2S)-3-(2-Hydroxy-2-(3-(pyridin-2-yl-(2,2,2-trichlorethoxycarbonyl)-amino)-propyl)-4-oxo-chroman-6-yl)-2-(2,2-dimethylpropyl)oxycarbonylamino-propionsäureethylester ("BC").

130 mg (0.18 mmol) "BC" werden in 5 ml Dichlormethan und 0.5 ml Trifluoressigsäure bei RT 15 h gerührt. Die Lösung wird anschließend eingeengt und der Rückstand an Kieselgel chromatographiert.
Ausbeute: 55 mg (2S)-3-(2-(3-((Pyridin-2-yl)-(2,2,2-trichlorethoxycarbonyl)-amino)-propyl)-4-oxo-4*H*-chromen-6-yl)-2-(2,2-dimethylpropyl)oxycarbonylamino-propionsäureethylester ("BD") FAB 686.

Die Abspaltung der TROC-Gruppe von "BD" erfolgt analog "Al" und liefert nach Aufarbeitung 40 mg (2*S*)-3-{2-[3-(Pyridin-2-ylamino)propyl]-4-oxo-4*H-*chromen-6-yl}-2-(2,2-dimethyl-propoxycarboxamido)-propionsäureethylester ("BE").

40 mg (78 µmol) "BE" werden in 2 ml Dioxan mit 1 ml 1 N HCl 60 h bei 70°C gerührt. Nach dem Einengen wird der Rückstand durch präp. HPLC an RP-18 Kieselgel gereinigt.
Ausbeute: 20 mg (2S)-3-{2-[3-(Pyridin-2-ylamino)propyl]-4-oxo-4*H-*chromen-6-yl}-2-(2,2-dimethyl-propoxycarboxamido)-propionsäure ("BF") als weißes, amorphes Pulver nach Gefriertrocknung, F. 80-85°, FAB 482.

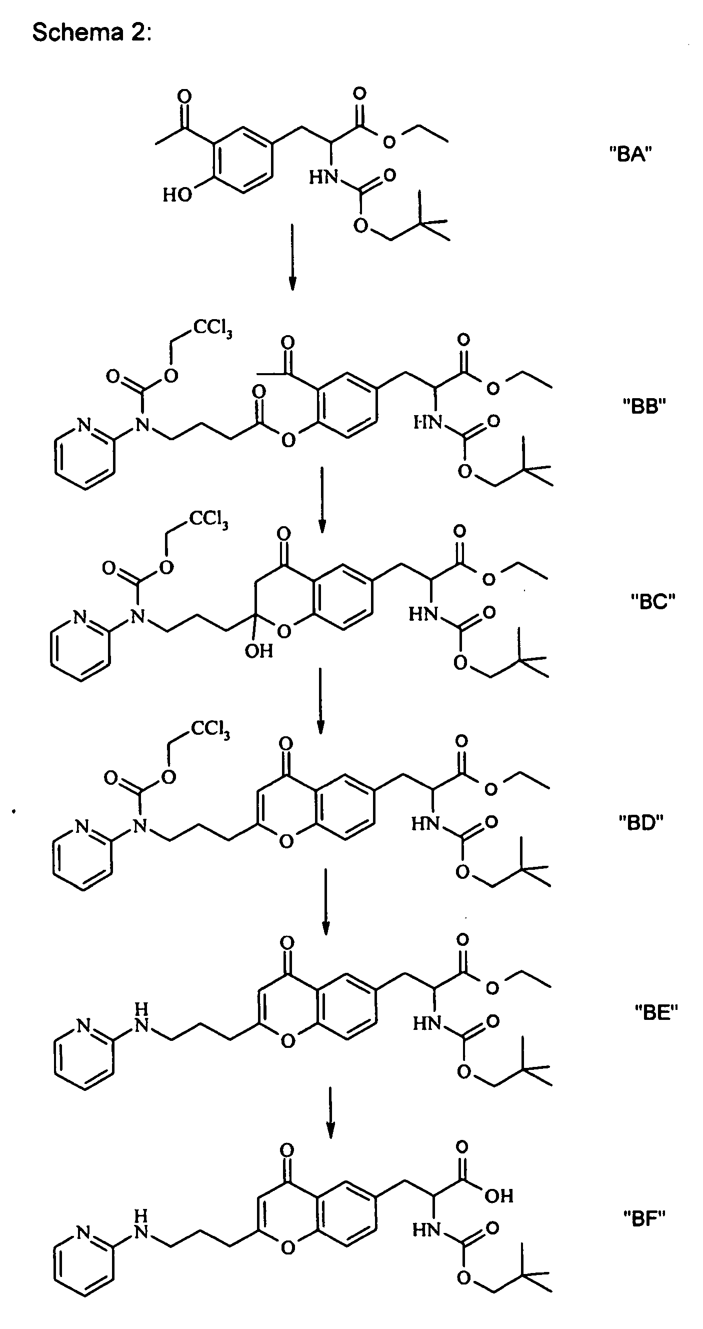

### Beispiel 3

### (2S)-3-{2-[3-(1H-Benzimidazol-2-ylamino)propyl]-4-oxo-4H-chromen-6-yl}-2-(2,2-dimethyl-propoxycarboxamido)-propionsäure:

Die Synthese der Verbindung erfolgt z.B. wie in Schema 3 angegeben.

6.5 g (17.8 mmol) "AD" werden analog Beispiel 1 mit 5.2 g (35.6 mmol) 4-Acetoxy-buttersäure in Gegenwart von 7.5 g (39.1 mmol) EDCI und 5.9 ml (53.6 mmol) NMP in 100 ml DMF umgesetzt. Das Produkt wird durch Chromatographie an Kieselgel mit Toluol/Aceton 6:1 als Eluenten gereinigt.
Ausbeute: 7.7 g 4-Acetoxy-buttersäure-(2-acetyl-4-(2-carboxyethyl-2-(2,2-dimethylpropyl)oxycarbonylamino-ethyl)-phenylester ("CA") als farbloses Öl, FAB 494.

Entsprechend Beispiel 1 werden 7.7 g (15.7 mmol) "CA" mit 489 mg (16.3 mmol) NaH (80% in Mineralöl) in 200 ml THF 16 h bei RT umgesetzt und aufgearbeitet.
Ausbeute: 7.2 g (2S)-3-(2-Hydroxy-2-(3-acetoxy-propyl)-4-oxo-chroman-6-yl)-2-(2,2-dimethylpropyl)oxycarbonylamino-propionsäureethylester ("CB") als Rohprodukt, das ohne Reinigung weiter umgesetzt wird.

Analog Beispiel 1 verläuft die Dehydratisierung von 7.2 g (15.7 mmol) "CB" mit 18 ml Trifluoressigsäure in 180 ml Dichlormethan in 48 h bei RT. Das nach Einengen der Reaktionslösung erhaltene Rohprodukt wird getrocknet und direkt weiter umgesetzt.
Ausbeute: 7.0 g (2S)-3-(2-(3-Acetoxy-propyl)-4-oxo-4*H*-chromen-6-yl)-2-(2,2-dimethylpropyl)oxycarbonylamino-propionsäureethylester ("CC") als farbloses Öl.

7.0 g (14.7 mmol) "CC" werden in 200 ml wasserfreiem Ethanol mit 1.9 g (28 mmol) Natiumethylat 1 h bei RT gerührt. Anschließend neutralisiert man mit saurem lonentauscher, engt die Lösung zum Rückstand ein und chromatographiert an Kieselgel mit Toluol/Aceton 2:1.
Ausbeute: 2.4 g (2S)-3-(2-(3-Hydroxy-propyl)-4-oxo-4*H*-chromen-6-yl)-2-(2,2-dimethylpropyl)oxycarbonylamino-propionsäureethylester ("CD"), FAB 434.

500 mg (1.15 mmol) "CD" werden in 20 ml Dichlormethan gelöst und 1.5 h bei RT mit 370 mg (1.73 mmol) Pyridiniumchlorochromat oxidiert. Die Reaktionslösung wird über 30 g Kieselgel filtriert, der Filterkuchen mit Ethylacetat nachgewaschen und die Lösung eingeengt. Das Rohprodukt wird ohne weitere Reinigung umgesetzt.
Ausbeute: 392 mg (2S)-3-(2-(3-Oxopropyl)-4-oxo-4*H*-chromen-6-yl)-2-(2,2-dimethylpropyl)oxycarbonylamino-propionsäureethylester ("CE").

Das Rohprodukt "CE" (100 mg, 0.23 mmol) wird in 10 ml Pyridin gelöst und in Gegenwart von 0.13 ml (0.93 mmol) Triethylamin mit 33 mg (0.25 mmol) 2-Amino-benzimidazol umgesetzt. Nach beendeter Reaktion (3 h bei RT) gibt man 18 mg (0.46 mmol) Natriumborhydrid hinzu und rührt weitere 3 h bei RT. Anschließend neutralisiert man mit verd. Essigsäure, engt die Lösung ein und reinigt den Rückstand durch präp. HPLC an RP-18.
Ausbeute: 64 mg (2S)-3-{2-[3-(1*H*-Benzimidazol-2-ylamino)propyl]-4-oxo-4*H*-chromen-6-yl}-2-(2,2-dimethyl-propoxycarboxamido)-propionsäureethylester ("CF") als farbloses amorphes Pulver nach Gefriertrocknung, FAB 549.

50 mg (0.09 mmol) "CF" werden in 2 ml Dioxan mit 1 ml 1N HCl 12 h bei 80°C gerührt und anschließend eingeengt.
Ausbeute: 45 mg (2S)-3-{2-[3-(1*H*-Benzimidazol-2-ylamino)propyl]-4-oxo-4*H*-chromen-6-yl}-2-(2,2-dimethyl-propoxycarboxamido)-propionsäure ("CG"), FAB 521.

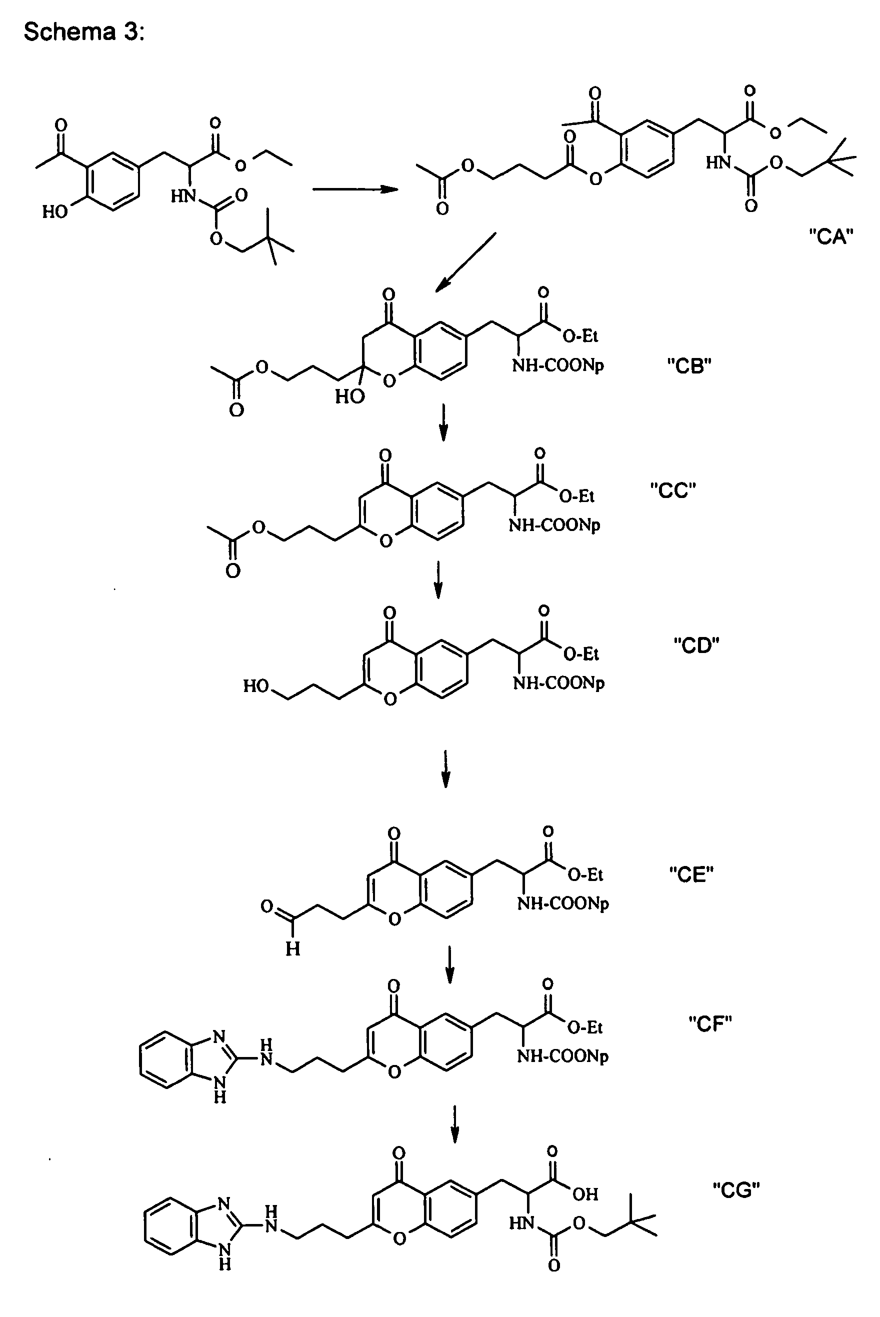

### Beispiel 8

Analog den Beispielen 1, 2 und 3 erhält man nachstehende Sulfonamidderivate
(2S)-3-{2-[3-(Pyridin-2-ylamino)propyl]-4-oxo-4*H*-chromen-6-yl}-2-butylsulfonamido-propionsäure,
(2S)-3-{2-[3-(Pyridin-2-ylamino)propyl]-4-oxo-4*H*-chromen-6-yl}-2-benzylsulfonamido-propionsäure.

Die nachfolgenden Beispiele betreffen pharmazeutische Zubereitungen:

### Beispiel A: Injektionsgläser

Eine Lösung von 100 g eines erfindungsgemäßen Wirkstoffes und 5 g Dinatriumhydrogenphosphat wird in 3 l zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines erfindungsgemäßen Wirkstoffes mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines erfindungsgemäßen Wirkstoffes, 9,38 g NaH₂PO₄· 2 H₂O, 28,48 g Na₂HPO₄ ·12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines erfindungsgemäßen Wirkstoffes mit 99,5 g Vaseline unter aseptischen Bedingungen.

### Beispiel E: Tabletten

Ein Gemisch von 1 kg Wirkstoff, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff werden in üblicher Weise in Hartgelatine kapseln gefüllt, so daß jede Kapsel 20 mg des Wirkstoffs enthält.

### Beispiel H: Ampullen

Eine Lösung von 1 kg Wirkstoff in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

### Beispiel I: Inhalations-Spray

Man löst 14 g Wirkstoff in 10 l isotonischer NaCl-Lösung und füllt die Lösung in handelsübliche Sprühgefäße mit Pump-Mechanismus. Die Lösung kann in Mund oder Nase gesprüht werden. Ein Sprühstoß (etwa 0,1 ml) entspricht einer Dosis von etwa 0,14 mg.

## Patentansprüche

1. Verbindungen ausgewählt aus der Gruppe
a) (2S)-3-{2-[3-(1*H*-Imidazol-2-ylamino)propyl]-4-oxo-4H-chromen-6-yl}-2-(2,2-dimethyl-propoxycarboxamido)-propionsäure-ethylester,
b) (2S)-3-{2-[3-(1*H*-Imidazol-2-ylamino)propyl]-4-oxo-4*H*-chromen-6-yl}-2-(2,2-dimethyl-propoxycarboxamido)-propionsäure,
c) (2S)-3-{2-[3-(Pyridin-2-ylamino)propyl]-4-oxo-4*H*-chromen-6-yl}-2-(2,2-dimethyl-propoxycarboxamido)-propionsäure,
d) (2S)-3-{2-[3-(1*H*-Benzimidazol-2-ylamino)propyl]-4-oxo-4*H*-chromen-6-yl}-2-(2,2-dimethyl-propoxycarboxamido)-propionsäureethylester,
e) (2S)-3-{2-[3-(1*H*-Benzimidazol-2-ylamino)propyl]-4-oxo-4*H*-chromen-6-yl}-2-(2,2-dimethyl-propoxycarboxamido)-propionsäure,
f) (2S)-3-{2-[3-(Pyridin-2-ylamino)propyl]-4-oxo-4*H*-chromen-6-yl}-2-butylsulfonamido-propionsäure,
g) (2S)-3-{2-[3-(Pyridin-2-ylamino)propyl]-4-oxo-4*H*-chromen-6-yl}-2-benzylsulfonamido-propionsäure,
sowie deren physiologisch unbedenklichen Salze und Solvate.

2. Verwendung von Verbindungen nach Anspruch 1 und ihrer physiologisch unbedenklichen Salze und Solvate zur Herstellung eines Arzneimittels zur Bekämpfung von pathologisch angiogenen Erkrankungen, Thrombosen, Herzinfarkt, koronaren Herzerkrankungen, Arteriosklerose, Tumoren, Osteoporose und rheumatischer Arthritis.

3. Pharmazeutische Zubereitung, **gekennzeichnet durch** einen Gehalt an mindestens einer Verbindung nach Anspruch 1 und/oder einem ihrer physiologisch unbedenklichen Salze oder Solvate.

4. Verfahren zur Herstellung einer pharmazeutischen Zubereitung, **dadurch gekennzeichnet, daß** man eine Verbindung nach Anspruch 1 und/oder eines ihrer physiologischen unbedenklichen Salze oder Solvate zusammen mit mindestens einem festen, flüssigen oder halbflüssigen Träger- oder Hilfsstoff in eine geeignete Dosierungsform bringt.

5. Verwendung von Verbindungen nach Anspruch 1 und ihrer physiologisch unbedenklichen Salze und Solvate zur Herstellung eines Arzneimittels zur Verwendung als αᵥ-Integrin-Inhibitor.

## Claims

1. Compounds selected from the group
a) ethyl (2S)-3-{2-[3-(1*H*-imidazol-2-ylamino)propyl}-4-oxo-4*H*-chromen-6-yl}-2-(2,2-dimethylpropoxycarboxamido)propionate,
b) (2S)-3-{2-[3-(1*H*-imidazol-2-ylamino)propyl]-4-oxo-4*H*-chromen-6-yl}-2-(2,2-dimethylpropoxycarboxamido)propionic acid,
c) (2S)-3-{2-[3-(pyridin-2-ylamino)propyl]-4-oxo-4*H*-chromen-6-yl}-2-(2,2-dimethylpropoxycarboxamido)propionic acid,
d) ethyl (2S)-3-{2-[3-(1*H*-benzimidazol-2-ylamino)propyl]-4-oxo-4*H-*chromen-6-yl}-2-(2,2-dimethylpropoxycarboxamido)propionate,
e) (2S)-3-{2-[3-(1*H*-benzimidazol-2-ylamino)propyl]-4-oxo-4*H*-chromen-6-yl}-2-(2,2-dimethylpropoxycarboxamido)propionic acid,
f) (2S)-3-{2-[3-(pyridin-2-ylamino)propyl]-4-oxo-4*H*-chromen-6-yl}-2-butylsulfonamidopropionic acid,
g) (2S)-3-{2-[3-(pyridin-2-ylamino)propyl]-4-oxo-4*H*-chromen-6-yl}-2-benzylsulfonamidopropionic acid,
and physiologically acceptable salts and solvates thereof.

2. Use of compounds according to Claim 1 and physiologically acceptable salts and solvates thereof for the preparation of a medicament for combating pathologically angiogenic diseases, thromboses, cardiac infarction, coronary heart diseases, arteriosclerosis, tumours, osteoporosis and rheumatic arthritis.

3. Pharmaceutical composition, **characterised by** a content of at least one compound according to Claim 1 and/or one of its physiologically acceptable salts or solvates.

4. Process for the preparation of a pharmaceutical composition, **characterised in that** a compound according to Claim 1 and/or one of its physiologically acceptable salts or solvates is brought into a suitable dosage form together with at least one solid, liquid or semi-liquid excipient or adjuvant.

5. Use of compounds according to Claim 1 and physiologically acceptable salts and solvates thereof for the preparation of a medicament for use as αᵥ integrin inhibitor.

## Revendications

1. Composés choisis parmi le groupe constitué par
a) le (2S)-3-{2-[3-(1*H*-imidazol-2-ylamino)propyl}-4-oxo-4*H*-chromén-6-yl}-2-(2,2-diméthylpropoxycarboxamido)propionate d'éthyle,
b) l'acide (2S)-3-{2-[3-(1*H*-imidazol-2-ylamino)propyl]-4-oxo-4*H*-chromén-6-yl}-2-(2,2-diméthylpropoxycarboxamido)propionique,
c) l'acide (2S)-3-{2-[3-(pyridin-2-ylamino)propyl]-4-oxo-4*H*-chromén-6-yl}-2-(2,2-diméthylpropoxycarboxamido)propionique,
d) le (2S)-3-{2-[3-(1*H*-benzimidazol-2-ylamino)propyl]-4-oxo-4*H*-chromén-6-yl}-2-(2,2-diméthylpropoxycarboxamido)propionate d'éthyle,
e) l'acide (2S)-3-{2-[3-(1*H*-benzimidazol-2-ylamino)propyl]-4-oxo-4*H-*chromén-6-yl}-2-(2,2-diméthylpropoxycarboxamido)propionique,
f) l'acide (2S)-3-{2-[3-(pyridin-2-ylamino)propyl]-4-oxo-4*H*-chromén-6-yl}-2-butylsulfonamidopropionique,
g) l'acide (2S)-3-{2-[3-(pyridin-2-ylamino)propyl]-4-oxo-4*H*-chromén-6-yl}-2-benzylsulfonamidopropionique,
et les sels et solvats physiologiquement acceptables de ceux-ci.

2. Utilisation de composés selon la revendication 1 et de sels et solvats physiologiquement acceptables de ceux-ci pour la préparation d'un médicament destiné à lutter contre les maladies pathologiquement angiogènes, les thromboses, l'infarctus du myocarde, les maladies coronariennes, l'artériosclérose, les tumeurs, l'ostéoporose et la polyarthrite rhumatoïde.

3. Composition pharmaceutique, **caractérisée par** une teneur en au moins un composé selon la revendication 1 et/ou en l'un de ses sels ou solvats physiologiquement acceptables.

4. Procédé de préparation d'une composition pharmaceutique, **caractérisé en ce qu'**un composé selon la revendication 1 et/ou l'un de ses sels ou solvats physiologiquement acceptables est mis sous une forme de dosage convenable conjointement avec au moins un excipient ou adjuvant solide, liquide ou semi-liquide.

5. Utilisation de composés selon la revendication 1 et de sels et solvats physiologiquement acceptables de ceux-ci pour la préparation d'un médicament destiné à une utilisation comme inhibiteur de l'intégrine αᵥ.
